# EUROPEAN PATENT APPLICATION

(11) **EP 3 156 502 A1**
(43) Date of publication of application: **19.04.2017**
(21) Application number: 15806729.8
(22) Date of filing: 30.03.2015
(51) Int. Cl.: C12Q 1/68

(54) **EVALUATION METHOD FOR EVALUATING THE LIKELIHOOD OF BREAST CANCER**

(30) Priority: 12.06.2014 ES 201430901
(71) Applicant: Sistemas Genómicos, S.L., 46980 Paterna (Valencia) (ES)
(72) Inventor: SAUS CANO, Lorena, 46980 Paterna (Valencia) (ES); BALLESTER DE MATIAS, Alida, 46980 Paterna (Valencia) (ES); TRIVIÑO PARDO, Juan Carlos, E-46980 Paterna (Valencia) (ES); CABO DÍEZ, Miguel, 46980 Paterna (Valencia) (ES); RODRÍGUEZ CRUZ, Oscar, 46980 Paterna (Valencia) (ES)
(74) Representative: Álvarez López, Sonia
(86) International application number: PCT/ES2015/070245
(87) International publication number: WO 2015/189444

(57) **Abstract**

The invention relates to a method for evaluating the likelihood of a person having breast cancer, the method including detecting and quantifying specific microRNAs in serum or plasma, the expression of which is related to the presence of a breast tumour. The invention also relates to the use of specific microRNAs, the expression of which is related to the presence of a breast tumour.

LISTADO DE SECUENCIAS

<110> Sistemas Genómicos

<120> MÉTODO DE EVALUACIÓN PARA EVALUAR UNA POSIBILIDAD DE CÁNCER DE MAMA

<160> 5

<170> BiSSAP 1.0

<210> 1
<211> 23
<212> other RNA
<213> Homo sapiens

<220>
<221> source
<222> 1..23
<223> /mol_type="other RNA"
      /organism="Homo sapiens"

<400> 1
tgtagtgttt cctactttat gga                                              23

<210> 2
<211> 22
<212> other RNA
<213> Homo sapiens

<220>
<221> source
<222> 1..22
<223> /mol_type="other RNA"
      /organism="Homo sapiens"

<400> 2
gggagccagg aagtattgat gt                                               22

<210> 3
<211> 22
<212> other RNA
<213> Homo sapiens

<220>
<221> source
<222> 1..22
<223> /mol_type="other RNA"
      /organism="Homo sapiens"

<400> 3
tagcttatca gactgatgtt ga                                               22

<210> 4
<211> 22
<212> other RNA
<213> Homo sapiens

<220>
<221> source
<222> 1..22

<223> /mol_type="other RNA"
      /organism="Homo sapiens"

<400> 4
tccctgagac cctaacttgt ga                                                22


<210> 5
<211> 17
<212> other RNA
<213> Homo sapiens

<220>
<221> source
<222> 1..17
<223> /mol_type="other RNA"
      /organism="Homo sapiens"

<400> 5
   ggcgggtgcg ggggtgg                                                       17

## Description

The present invention relates to a method for evaluating the likelihood of a subject having breast cancer, the method including detecting and quantifying specific microRNAs in serum or plasma, the expression of which is related to the presence of a breast tumour. The invention also relates to the use of specific microRNAs, the expression of which is related to the presence of a breast tumour.

The present invention is framed within the field of the development of non-invasive early diagnostic methods for mammary neoplastic transformations, for example in order to complement habitual image diagnoses.
Breast cancer is one of the five most common types of cancer, and its high incidence is the main reason behind the intensive research being conducted, primarily focused on early detection and quick diagnosis. Within this context, the -omic sciences have made great progress in the search for specific molecular markers for the early detection and diagnosis of this type of cancer (Kim BMC Bioinformatics 2010). Amongst the molecular markers studied, certain microRNAs are being evaluated for their potential use as diagnostic and prognostic markers (Shi, Cancer Treat Rev 2009).

MicroRNAs (miRNAs) are small single-stranded noncoding RNA molecules that modulate gene expression, with a size ranging between eighteen and twenty-five nucleotides (Aoife L., Clin Can Res, 2008; Bartels, Clin Chem 2009; Zoon, Exp. Rev. Mol. Diagn. 2009). miRNAs act as post-transcriptional regulators and control numerous cellular processes, such as development and differentiation, cell cycle and apoptosis. Thus, they are expressed in the cell nucleus of eukaryotic cells, in both intragenic and intergenic regions, and may regulate gene expression at the post-transcriptional level, blocking the translation and/or inducing the degradation of messenger RNA (mRNA) by binding to the complementary 3' untranslated region (UTR) thereof. Various recent studies suggest that microRNAs are involved in the pathogenesis of all types of cancer in humans (MICRORNAS: NUEVOS MARCADORES DE INTERÉS EN ONCOLOGÍA, Ginés Luengo Gil, Haematology and Medical Oncology Service, Morales Meseguer University Hospital, Regional Haemodonation Centre and Department of Internal Medicine, University of Murcia, REVISTA EUBACTERIA (APRIL 2012) No. 28 / ISSN-1697-0071).

Thus far, approximately 1,000 miRNAs have been identified in humans, of which approximately 200 are related to tumoural processes, although it has been suggested that there are tens of thousands. The appearance and continuous development of massive sequencing techniques has been essential for the discovery and identification of this type of molecules, since their small size and their low concentration in eukaryotic cells makes it difficult to detect them using traditional molecular techniques (Nygaard, BMC Med Gen, 2009). Moreover, a key aspect of the biogenesis of these small RNA molecules that makes them attractive as molecular biomarkers is the fact that they remain stable in serum and plasma, thus allowing for their detection in both specific tissues and this type of fluids (Lodes, PLoS One, 2009).

In the standard nomenclature system, microRNAs (miRBase) are assigned a name composed of 4 parts, of the type mmu-miR-375-5p. The first three letters indicate the species, for example hsa (homo sapiens) for humans. A lower-case microRNA name (for example, hsa-mir-22) refers to the microRNA gene or the pre-microRNA, whereas the mature microRNA is associated with the term miR (as in hsa-miR-22-5p). The numeric reference associated with the name is sequentially assigned, whereas the last alphanumeric reference refers to the arm (5p or 3p, respectively 5' or 3').

Approximately 20-30 specific differentially expressed miRNAs have been characterised in breast cancer. The best characterised examples include miR-21, which has an oncogenic character, miR-29A, miR-17-5p, miR-26A, miR-181A, miR-22, miR196A-2, etc. (Maillot, Cancer Res 2009; Bourguignon, JBC 2009; Wu, Journal of Biomedicine and Biotechnology, 2011). Currently, the expression patterns of these specific miRNAs are being used to differentiate the healthy breast tissue adjacent to the tumoural tissue, in order to classify the different types of tumours and make progress in prognosis (Lowery, Clin Can Res 2008; Enerly, PLoS One 2011, Rothe, PLoS One 2011).

Breast cancers may be classified into molecular subtypes on the basis of different genetic expression profiles. Perou et al. (Perou, C.M., Sorlie, T., Eisen, M.B., van de Rijn, M., Jeffrey, S.S., Rees, C.A., Pollack, J.R., Ross, D.T., Johnsen, H., Akslen, L.A., et al. (2000), Molecular portraits of human breast tumours, Nature 406, 747-752) revealed the molecular stratification of breast tumours into reproducible molecular subtypes of breast cancer with different biological characteristics, clinical results and responses to therapy. The classification includes two groups based on the gene expression of the oestrogen receptor (ER): ER+ (luminal A, luminal B, luminal C) and ER- (Her-2, basal type or triple-negative, normal type). These subgroups have been associated with different evolutions of the disease, which indicates the existence of a biological basis for the clinical heterogeneity of breast cancer.

Thus, the identification of specific microRNAs for the different breast tumours may contribute, in an effective and probably more precise manner, to the diagnosis and prognosis of the disease.
Therefore, given the potential of this type of markers, active research has been conducted on the process whereby the specific miRNAs expressed in the malignant tumour cells are released into the bloodstream. In the case of some miRNAs, such as miR-451 and miR-1246, it has been demonstrated that they are expressed in malignant mammary epithelial cells and selectively released into the circulation, whereas they are intracellularly retained in healthy epithelial cells (Pigati, PLoS One, 2010). Recent studies show that these specific miRNAs may be detected by means of amplification reactions starting from peripheral blood, since these molecules have a small size and are very stable (Zhao, Cancer PLoS One 2010).
For example, document WO2014023797 discloses an ex vivo method for determining whether or not a patient's tumour cells are or may be resistant to a medicament that modulates the binding of a steroid hormone to its receptor.

The method described in this document makes it possible to select which type of hormone treatment is the most suitable in each case for certain types of breast cancer.

US 20120214864 relates to a method for identifying a triple-negative tumour in a sample from a subject, in particular tumoural tissue, wherein a reduced level of certain miRNAs, as compared to those corresponding to a luminal tumour, indicate that the subject is suffering from triple-negative cancer.
The present invention provides a method for evaluating the likelihood of a subject having breast cancer, wherein the method includes detecting and quantifying, in a serum or plasma sample of the subject in question, the expression of at least one specific microRNA selected from hsa-miR-142-3p (UGUAGUGUUUCCUACUUUAUGGA, SEQ ID NO: 1, Access No. MIMAT0000434), hsa-miR-505-5p (GGGAGCCAGGAAGUAUUGAUGU, SEQ ID NO: 2, Access No. MIMAT0004776), hsa-miR-21-5p (UAGCUUAUCAGACUGAUGUUGA, SEQ ID NO: 3, Access No. MIMAT0000076), hsa-miR-125b-5p (UCCCUGAGACCCUAACUUGUGA, SEQ ID NO: 4, Access No. MIMAT0000423) and hsa-miR-3656 (GGCGGGUGCGGGGGUGG, SEQ ID NO: 5, Access No. MIMAT0018076), and/or combinations thereof, specific to the Spanish population, the expression whereof is related to the presence of a breast tumour; comparing the expression, by means of statistical analysis, to a reference value obtained from a healthy subject, in order to determine whether or not there is overexpression; and evaluating the likelihood of breast cancer on the basis of a possible tumoural presence when at least one miRNA is overexpressed in the sample with a value greater than an optimal cut-off value.
Preferably, in the method described above, a combination of hsa-miR-125b-5p (UCCCUGAGACCCUAACUUGUGA, SEQ ID NO: 4, Access No. MIMAT0000423) and hsa-miR-3656 (GGCGGGUGCGGGGGUGG, SEQ ID NO: 5, Access No. MIMAT0018076) is used.

The invention also relates to the use of the aforementioned specific microRNAs or combinations thereof, the expression of which is related to the presence of a breast tumour, including any tumour subtype (Her2+, Luminal A and B, and Triple-negative), in a method such as the one described.
The following figures and examples are supplied solely for illustrative purposes, and do not limit the scope of the invention.

In the figures:
- fig. 1: is a methodological diagram of RNA extraction from breast tissue and the identification of differentially expressed miRNAs by means of a microarray platform;
- fig. 2: shows the statistical results for the difference in expression of the differentially expressed miRNAs obtained in the study group and the control group;
- fig. 3: shows the statistical results for the difference in expression of the miRNAs in each of the tumour subtypes studied;
- fig. 4: is a statistical study of differential expression in plasma, wherein miRNA1 corresponds to SEQ ID NO: 1, miRNA2 corresponds to SEQ ID NO: 2, miRNA3 corresponds to SEQ ID NO: 3, miRNA4 corresponds to SEQ ID NO: 4, and miRNA5 corresponds to SEQ ID NO: 5.

### 1. Methodology applied

### Sample selection

Samples of paraffin-embedded tissue were collected from patients diagnosed with Triple-negative, luminal A, luminal B and Her2 tumours, for the study group, and healthy breast tissue was collected for the control group, as shown in the following Table 1:

**Table 1**

| Study group | |
|---|---|
| Type of tumour | Number of patients |
| Triple-negative | 22 |
| Luminal A | 19 |
| Luminal B | 15 |
| Her2 | 15 |
| Control group | Number of samples |
| Healthy breast tissue | 14 |

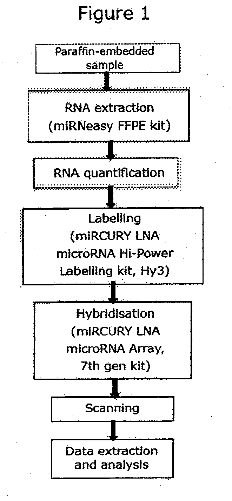

All these samples were processed in order to identify the differentially expressed miRNAs, as shown in Fig. 1. In the case of the paraffin-embedded tissue samples, a microdissection technique was applied to the paraffin-embedded cuts in order to specifically extract RNA from the embedded tissue, and thus prevent background interferences and noise in the results for the expression study.

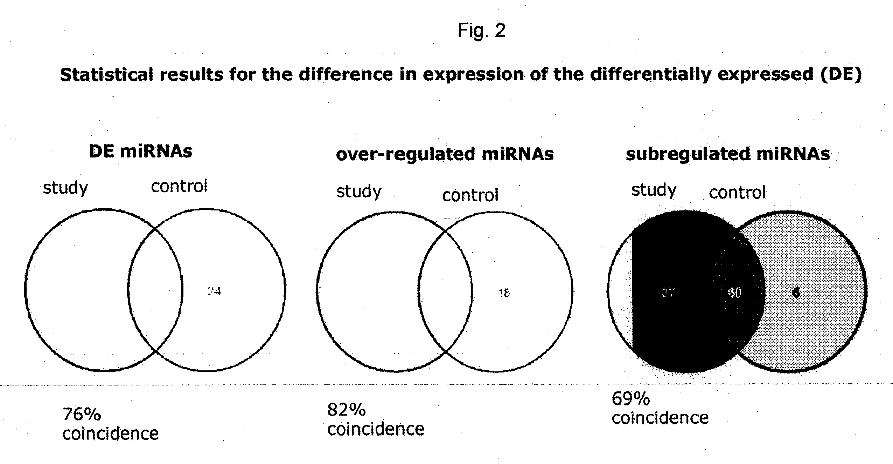

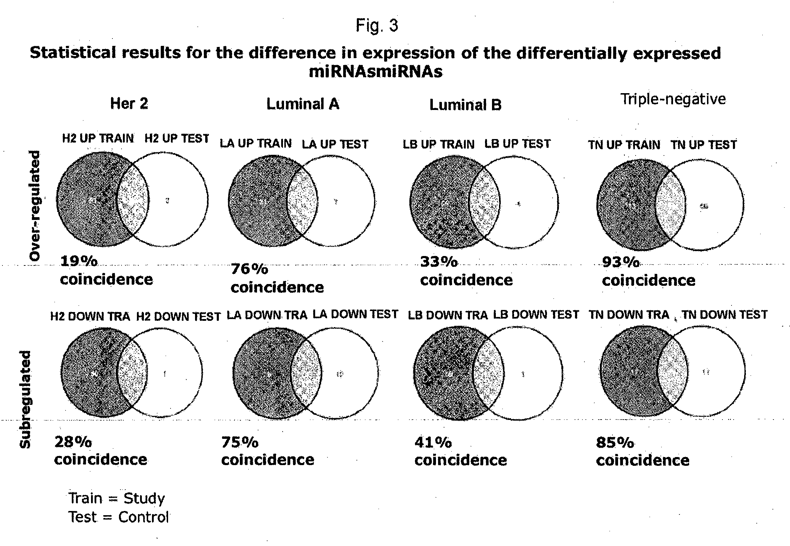

Analysis of the results and selection of candidate miRNAs
The results for the different expression profiles of the samples hybridised on the array platform were filtered and statistically analysed. This statistical analysis basically involves processing the raw data obtained from the array platform in order to obtain a list of differentially expressed miRNAs, in a greater or lesser proportion, which are statistically significant as compared to the control group. The objective of this analysis is to study the expression of the miRNAs detected by the microarray platform used, in this case 1,926 (miRCURY LNA microRNA Array, 7th gen), in order to analyse the different expression patterns of each type of tumour, where the expression value of the subregulated and overexpressed miRNAs in each of them is identified by means of pipeline analysis and statistical algorithms. In order to filter and normalise the data obtained, those miRNAs with an expression lower than a threshold value V < 0.07 are eliminated. The results obtained are shown in the following Table 2 (see Figs. 2 and 3):

**Table 2**

| Group | Differentially expressed miRNAs | Subregulated | Overexpressed |
|---|---|---|---|
| Study | 191 | 104 | 87 |
| Control | 169 | 103 | 66 |

The selection of the differentially expressed miRNAs in each subtype supplies a specific genetic signature for each type of tumour.
Using the selection of miRNAs obtained, work was performed on the entire group of tumours in order to obtain a signature indicating the presence of any breast tumour. As a result of this analysis, a group of 27 miRNAs were obtained which present very suitable precision, sensitivity and specificity parameters, 98%, 100% and 83%, respectively.

### 2. Methodology applied

Complete blood, plasma and serum samples were obtained (in specific tubes for expression studies in blood) and processed in order to obtain RNA fractions wherein the miRNAs of interest were present. The plasma samples were selected, since they made it possible to extract the miRNA fraction using a suitable purification kit. Subsequently, the expression of the selected miRNAs was analysed by means of the processes described above, in order to obtain a group of potential statistically robust, significant miRNAs.

The set of 27 miRNAs was subjected to prioritisation on the basis of the probes available for the detection thereof in the qRT-PCR miRCURY LNA system (quantitative reverse PCR). This first reading group was based on 18 markers for which pre-designed validated probes for the expression study were found.

In order to detect the miRNAs, the RNA was retrotranscribed to cDNA and the amplification parameters were adjusted following the supplier's instructions.
The detection of differences in expression in the miRNAs in the samples of paraffin-embedded tumours was also tested, since the change in methodology from the array platform to detection by means of qRT-PCR could affect the detection of some of them. The following results were obtained from the amplification reactions in the samples of paraffin-embedded tumours: of the 18 miRNAs selected, 8 could not be amplified by means of qRT-PCR and, of the 10 remaining ones, 9 were differentially expressed. These 9 differentially expressed miRNAs were then detected in the plasma samples by means of qRT-PCR; the expression was detected for 8 of them and, of these, five were differentially expressed.

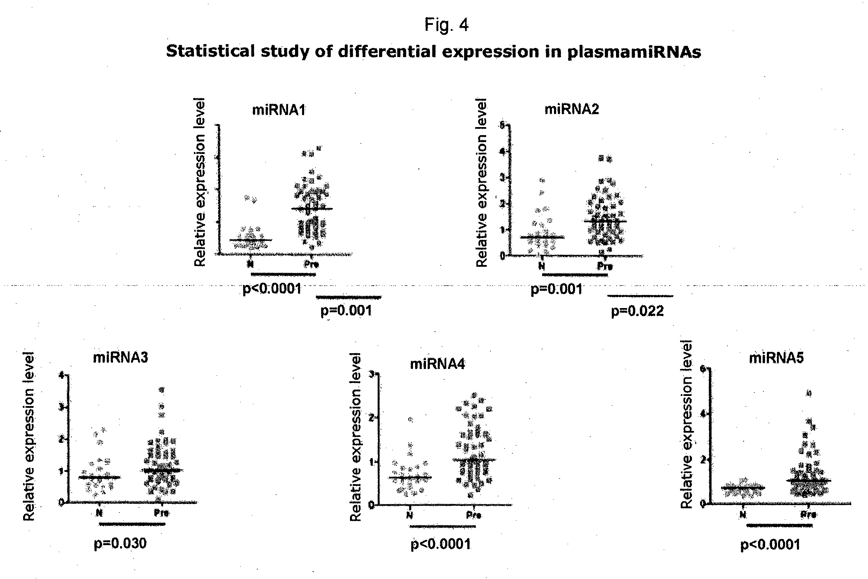

A statistical significance analysis was performed with these five miRNAs, by studying the results for the difference in expression of the five miRNAs in the complete series of samples, and the results shown in Fig. 4 were obtained. As may be clearly observed in this figure, the relative overexpression level of the five selected miRNAs indicates that they are significantly expressed in the samples obtained from subjects with breast tumours ("Pre" in Figure 4). In all cases, the significance value used to assume that the expression of the relevant miRNA was significantly different was < 0.05.

On the basis of the results obtained, the cut-off value was calculated for each of the selected miRNAs, this value being understood to be the limit value that differentiates between positive and negative, i.e. the optimal value for each miRNA which divides the results in such a way that there are more overexpressed miRNAs in the tumour samples above or below said value, and the opposite occurs in the case of healthy samples.

### Example

In order to evaluate the likelihood of breast cancer in plasma obtained from a particular subject, the expression of the selected microRNAs is quantified by means of qPCR, as described above, in each sample; in this example, hsa-miR-142-3p, hsa-miR-505-5p, hsa-miR-21-5p, hsa-miR-125b-5p and hsa-miR-3656, and the combination hsa-miR-125b-5p + hsa-miR-3656; those chosen as a reference for the subject were also quantified, in this example hsa-miR103a-3p and hsa-miR-16-5p, since they have a very constant expression level in the general population. For each miRNA, the normalised expression value is calculated by subtracting the value for the reference miRNA from the value for the miRNA of interest in each sample. Below we compare the expression by means of statistical analysis, as described above, with a control reference value for a healthy subject, in order to determine whether or not there is overexpression. Finally, the likelihood of breast cancer is evaluated on the basis of a possible tumoural presence, assuming that the overexpression of the miRNA in question indicates the presence of the tumour if the resulting value is greater than the optimal cut-off value in each case.

| miRNA | AUC (area under the curve) | Standard error | 95% confidence interval | p Value | Optimal cut-off | Sensitivity (%) | Specificity %) |
|---|---|---|---|---|---|---|---|
| miR-142-3p | 0.6509 | 0.06607 | 0.5214-0.7805 | 0.03004 | 0.9171 | 64 | 69 |
| miR-505-5p | 0.7333 | 0.06323 | 0.6094-0.8573 | 0.001012 | 0.9599 | 73 | 72 |
| miR-21-5p | 0.7781 | 0.05417 | 0.6719-0.8843 | < 0.0001 | 0.8896 | 67 | 81 |
| miR-125b-5p | 0.7956 | 0.04934 | 0.6988-0.8923 | < 0.0001 | 0.8599 | 70 | 92 |
| miR-3656 | 0.8938 | 0.03953 | 0.8163-0.9714 | < 0.0001 | 0.6310 | 90 | 81 |
| miR-3656 + miR-125b-5p | 0.9138 | 0.03353 | 0.8481-0.9796 | < 0.0001 | 0.5856 | 84 | 92 |

## Claims

1. Method for evaluating the likelihood of breast cancer in a subject, the method including:
i. detecting and quantifying, in a serum or plasma sample from the subject, the expression of at least one specific microRNA selected from:
hsa-mR-142-3p (UGUAGUGUUUCCUACUUUAUGGA, SEQ ID NO: 1),
hsa-miR-505-5p (GGGAGCCAGGAAGUAUUGAUGU, SEQ ID NO: 2),
hsa-miR-21-5p (UAGCUUAUCAGACUGAUGUUGA, SEQ ID NO: 3),
hsa-miR-125b-5p (UCCCUGAGACCCUAACUUGUGA, SEQ ID NO: 4), and
hsa-miR-3656 (GGCGGGUGCGGGGGUGG, SEQ ID NO: 5), and/or
any combination thereof, specific to the Spanish population;
ii. comparing the expression, by means of statistical analysis, to a reference value obtained from a healthy subject, in order to determine whether or not there is overexpression; and
iii. evaluating the likelihood of breast cancer on the basis of a possible tumoural presence when at least one miRNA or a combination of any of the miRNAs is overexpressed in the sample, with a value greater than an optimal cut-off value.

2. Method for evaluating the likelihood of breast cancer in a subject according to claim 1, wherein, in step i., the combination of hsa-miR-125b-5p, SEQ ID NO: 4, and hsa-miR-3656, SEQ ID NO: 5, is selected.

3. Use of at least one specific microRNA selected from hsa-miR-142-3p (UGUAGUGUUUCCUACUUUAUGGA, SEQ ID NO: 1), hsa-miR-505-5p (GGGAGCCAGGAAGUAUUGAUGU, SEQ ID NO: 2), hsa-miR-21-5p (UAGCUUAUCAGACUGAUGUUGA, SEQ ID NO: 3), hsa-miR-125b-5p (UCCCUGAGACCCUAACUUGUGA, SEQ ID NO: 4) and
hsa-miR-3656 (GGCGGGUGCGGGGGUGG, SEQ ID NO: 5) and/or any combinations thereof,
specific to the Spanish population, following a method according to claim 1.
